# NEUE EUROPÄISCHE PATENTSCHRIFT

(11) **EP 0 828 748 B2**
(45) Veröffentlichungstag und Bekanntmachung des Hinweises auf die Entscheidung über den Einspruch: **03.09.2003**
(45) Hinweis auf die Patenterteilung: 11.10.2000
(21) Anmeldenummer: 96917396.2
(22) Anmeldetag: 22.05.1996
(51) Int. Cl.: C07H 15/04

(54) **VERFAHREN ZUR HERSTELLUNG HELLFARBIGER, NIEDRIGVISKOSER ALKYLOLIGOGLYKOSIDE**
PROCESS FOR PRODUCING LIGHT-COLOURED, LOW-VISCOSITY ALKYL OLIGOGLYCOSIDES
PROCEDE DE PREPARATION D'ALKYLOLIGOGLUCOSIDES PEU VISQUEUX DE COULEUR CLAIRE

(30) Priorität: 31.05.1995 DE 19519889
(43) Veröffentlichungstag der Anmeldung: 18.03.1998
(73) Patentinhaber: Cognis Deutschland GmbH & Co. KG, 40589 Düsseldorf (DE)
(72) Erfinder: WEUTHEN, Manfred, D-42697 Solingen (DE)
(86) Internationale Anmeldenummer: EP9602192
(87) Internationale Veröffentlichungsnummer: WO96038459

(56) Entgegenhaltungen:
- WO-A-90/03216
- WO-A-92/14809
- WO-A-93/07249
- DE-A- 2 555 076
- DE-A- 4 142 592
- US-A- 4 898 934
- US-A- 4 990 605
- Plantaren 2000CS "provisional datasheet; May 1992"
- Plantaren Broschüre 2/1992 (eingegangen am 4/11/02)

## Beschreibung

### Gebiet der Erfindung

Die Erfindung betrifft ein Verfahren zur Herstellung niedrigviskoser Alkyloligoglucoside mit verbesserter Farbqualität, bei dem man zunächst definerte Mischungen lang- und kurzkettiger Alkyloliglykoside herstellt und diese dann in an sich bekannter Weise einer alkalischen Bleiche unterwirft.

### Stand der Technik

Alkyloligoglykoside, insbesondere Alkyloligoglucoside stellen nichtionische Tenside dar, die infolge ihrer ausgezeichneten Detergenseigenschaften und hohen ökotoxikologischen Verträglichkeit zunehmend an Bedeutung gewinnen. Herstellung und Verwendung dieser Stoffe sind gerade in letzter Zeit in einer Reihe von Übersichtsartikeln dargestellt worden, von denen stellvertretend die Veröffentlichungen von H.Hensen in **Skin Care Forum, 1, (Okt. 1992),** D.Balzer und N.Ripke in **Seifen-Öle-Fette-Wachse 118, 894(1992)** und B.Brancq in **Seifen-Öle-Fette-Wachse 118, 905 (1992)** genannt werden sollen.

Für den Einsatz in Handgeschirrspülmitteln und Haarshampoos kommen ganz überwiegend kürzerkettige Glykoside in Betracht, die sich von Alkoholen mit 8 bis 14 Kohlenstoffatomen ableiten. Längerkettige Glykoside mit etwa 16 bis 18 Kohlenstoffatomen eignen sich hingegen eher zur Herstellung von Waschmitteln oder als Emulgatoren für kosmetische und pharmazeutische Produkte **[EP-B1 0553241** (SEPPIC)]. Die Herstellung dieser langkettigen Produkte ist jedoch gegenüber den etablierten kürzerkettigen Glykosiden mit einer Reihe von Problemen verbunden. Zum einen kann der im Überschuß eingesetzte Fettalkohol wegen des hohen Siedepunktes nur mit erheblichem technischen Aufwand vom Wertprodukt abgetrennt werden - weswegen man ihn häufig im Produkt beläßt - zum anderen bilden die langkettigen Glucosiden selbst in starker wäßriger Verdünnung gelartige, inhomogene Mischungen, die sich schwer rühren lassen und auf die daher das an sich bekannte alkalischen Bleichverfahren für Glykoside nur mit mäßigem Erfolg angewendet werden kann.

Die DE 4 142 592 A1 betrifft ein Verfahren zur Bleiche Obesflächen aktiver Verbindungen durch Kombination von Persaverstoffverbindungen mit ausge-wählten Bleich boostern. Die Bleiche von jewischen langkettiger und kurzkettiger Alkylpolyglykoside im Verhältnis 99 : 1 bis 40 : 60 wird in dieser Druchschrift jedoch nicht beschrieben.

Die komplexe Aufgabe der Erfindung hat somit darin bestanden, ein Verfahren zu entwickeln, das gleichzeitig niedrigviskose und hellfarbige langkettige Alkyloliglykoside zur Verfügung stellt, ohne daß dabei die anwendungstechnischen Eigenschaften der Produkte, insbesondere das Waschvermögen und die Emulgierleistung nachteilig beeinflußt werden.

### Beschreibung der Erfindung

Gegenstand der Erfindung ist ein Verfahren zur Herstellung hellfarbiger, niedrigviskoser Alkyloligoglykoside, bei dem man Mischungen von
(a) langkettigen Alkyloligoglucosiden der Formel (I),

   **R**^{**1**}**O-[G]**_{**p**} **(I)**

   in der R¹ für einen Alkylrest mit 16 bis 22 Kohlenstoffatomen, G für einen Zuckerrest mit 5 oder 6 Kohlenstoffatomen und p für Zahlen von 1 bis 10 steht, und
(b) kurzkettigen Alkyloligoglucosiden der Formel (II),

   **R**^{**2**}**O-[G]**_{**p**} **(II)**

   in der R² für einen Alkylrest mit 8 bis 14 Kohlenstoffatomen, G für einen Zuckerrest mit 5 oder 6 Kohlenstoffatomen und p für Zahlen von 1 bis 10 steht, bei einem pH-Wert im Bereich von 8 bis 13 mit Peroxidverbindungen bleicht,
wobei die Komponenten (a) und (b) im Gewichtsverhältnis 99 : 1 bis 40 : 60 eingesetzt werden.

Überraschenderweise wurde gefunden, daß schon der Zusatz geringer Mengen kürzerkettiger Glucoside die Viskosität der längerkettigen Glucoside soweit herabsetzt, daß sie einer alkalischen Bleiche leicht zugänglich sind. Die Erfindung schließt die Erkenntnis ein, daß die anwendungstechnischen Eigenschaften längerkettiger Alkyloligoglucoside selbst durch Zusatz einer gleichen Menge kürzerkettiger Glucoside nicht nachteilig beeinflußt werden. Demzufolge werden die Komponenten (a) und (b) im Gewichtsverhältnis 99 : 1 bis 40 : 60, vorzugsweise 95 : 5 bis 60 : 40 uns insbesondere 90 : 10 bis 75 : 25 eingesetzt.

### Alkyloligoglykoside

Alkyloligoglykoside stellen bekannte Stoffe dar, die nach den einschlägigen Verfahren der präparativen organischen Chemie erhalten werden können. Stellvertretend für das umfangreiche Schrifttum sei hier auf die Schriften **EP-A1 0301298** und **WO 90/03977** verwiesen.

Die Alkyloligoglykoside können sich von Aldosen bzw. Ketosen mit 5 oder 6 Kohlenstoffatomen, vorzugsweise der Glucose ableiten. Die bevorzugten Alkyloligoglykoside sind somit Alkyloligoglucoside.

Die Indexzahl p in den allgemeinen Formeln (I) und (II) gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyloligoglykoside mit einem mittleren Oligomerisierungsgrad p von 1,1 bis 3,0 eingesetzt. Aus anwendungstechnischer Sicht sind solche Alkyloligoglykoside bevorzugt, deren Oligomerisierungsgrad kleiner als 1,7 ist und insbesondere zwischen 1,2 und 1,4 liegt.

Der Alkylrest R¹ kann sich von primären Alkoholen mit 16 bis 22 Kohlenstoffatomen ableiten. Typische Beispiele sind Cetylalkohol, Stearylalkohol und Behenylalkohol sowie deren technische Mischungen, beispielsweise auf Basis von gehärtetem Palmöl oder Rindertalg. Der Alkylrest R² kann sich von primären Alkoholen mit 8 bis 14 Kohlenstoffatomen ableiten. Typische Beispiele sind Caprylalkohol, Caprinalkohol, Undecylalkohol, Laurylalkohol und Myristylalkohol sowie deren technische Mischungen wie beispielsweise C_{8/10}-Vorlauffettalkohol oder technische Kokosfettalkoholschnitte.

### Peroxidverbindungen

Unter Peroxidverbindungen sind Stoffe zu verstehen, die über mindestens eine Sauerstoff-Sauerstoff-Bindung verfügen. Typische Beispiele sind Salze wie etwa Natriumperoxid, Bariumperoxid, Natriumperborat oder Natriumpercarbonat und organische Saäuren wie etwa Perameisensäure oder Peressigsäure. Vorzugsweise wird als Peroxidverbindung jedoch Wasserstoffperoxid in Form einer wäßrigen Lösung mit einem Aktivsubstanzgehalt im Bereich von 5 bis 50 und vorzugsweise 20 bis 40 Gew.-% eingesetzt. Die Einsatzmenge beträgt üblicherweise 0,5 bis 5 und vorzugsweise 1 bis 3 Gew.-% bezogen auf das Glykosid.

### Bleichverfahren

Üblicherweise werden die beiden Glykoside in Form wäßriger Lösungen bzw. Pasten mit einander vermischt und unter starker Scherung intensiv homogenisiert, dann unter Zugabe von Basen wie beispielsweise wäßriger Natriumhydroxidlösung und/oder Magnesiumoxid auf einen pH-Wert im Bereich von 8 bis 13, vorzugsweise 10 bis 12 eingestellt und schließlich bei einer Temperatur im Bereich von 50 bis 90 und vorzugsweise 70 bis 80°C mit der Peroxidverbindung behandelt. Die Reaktionszeit kann dabei 1 bis 48, vorzugsweise 12 bis 24 h betragen.

### Gewerbliche Anwendbarkeit

Die nach dem erfindungsgemäßen Verfahren erhältlichen Alkyloligoglykoside sind niedrigviskos und hellfarbig und eignen sich zur Herstellung von Waschmitteln sowie beispielsweise zusammen mit Fettalkoholen als selbstemulgierende Systeme für die Herstellung von O/W-Emulsionen.

Die folgenden Beispiele sollen den Gegenstand der Erfindung näher erläutern, ohne ihn darauf einzuschränken.

### Beispiele

### Vergleichsbeispiel V1

In einer 250-ml-Rührappartur mit Ankerrührer wurden 60 g Wasser vorgelegt, auf 80°C erhitzt, mit 40 g Hexadecyloligoglucosid (DP = 1,5) versetzt und über einen Zeitraum von 1 h gerührt. Das Produkt zeigte danach eine gelförmige Konsistenz und war nicht vollständig homogen. Die Mischung wurde im Anschluß zunächst mit 0,8 g 50 Gew.-%iger wäßriger Natriumhydroxidlösung und dann nach einer Rührzeit von 1 h mit 1,7 g 35 Gew.-%iger wäßriger Wasserstoffperoxidlösung versetzt. Danach wurde das Produkt weitere 24 h gerührt. Die Versuchsergebnisse sind in Tabelle 1 zusammengefaßt.

### Beispiel 1:

Analog Vergleichsbeispiel V1 wurde eine Lösung von 40 g Kokosalkyloligoglucosid (Plantaren^{(R)} APG 600, Henkel KGaA, Düsseldorf/FRG, 50 Gew.-%ig in Wasser) zusammen mit 30 g Wasser bei einer Temperatur von 80°C mit 30 g Hexadecyloligoglucosid (DP = 1,5) versetzt und 30 min homogenisiert. Es resultierte eine gut rührfähige Paste, welche bei 80°C zunächst mit 1,0 g 50 Gew.-%iger wäßriger Natriumhydroxidlösung und nach einer Rührzeit von 10 min mit 2,1 g 35 Gew.%iger Wasserstoffperoxidlösung versetzt wurde. Danach wurde das Produkt weitere 24 h gerührt. Die Versuchsergebnisse sind in Tabelle 1 zusammengefaßt.

### Beispiel 2:

Analog Vergleichsbeispiel 1 wurde eine Lösung von 30 g C_{8/10}-Alkyloligoglucosid (Plantaren^{(R)} APG 225, Henkel KGaA, Düsseldorf/FRG, 35 Gew.-%ig in Wasser) zusammen mit 40 g Wasser bei einer Temperatur von 80°C mit 30 g C_{16/18}-Alkyloligoglucosid (DP = 1,5) versetzt und 30 min homogenisiert. Es resultierte eine gut rührfähige Paste, welche bei 80°C zunächst mit 1,0 g 50 Gew.-%iger wäßriger Natriumhydroxidlösung und nach einer Rührzeit von 10 min mit 2,1 g 35 Gew.-%iger Wasserstoffperoxidlösung versetzt wurde. Danach wurde das Produkt weitere 24 h gerührt. Die Versuchsergebnisse sind in Tabelle 1 zusammengefaßt.

### Beispiele 3 bis 5:

Beispiel 2 wurde wiederholt, auf 30 g C_{16/18}-Alkyloligoglucosid jedoch 0,3, 0,9 bzw. 3 g C_{8/10}-Alkyloligoglucosid eingesetzt. Die Versuchsergebnisse sind in Tabelle 1 zusammengefaßt.

**Tabelle 1**

| Versuchsergebnisse | | | |
|---|---|---|---|
| **Bsp.** | **(a):(b)** | **Farbzahl n.Bleiche Gardner/2cm Küvet.** | **Viskosität (70°C) mPa*s** |
| V1 | 100:0 | 5 | 25.000 |
| 1 | 60 : 40 | 2 | 2.000 |
| 2 | 60 : 40 | 3 | 1.500 |
| 3 | 99 : 1 | 3 | 9.000 |
| 4 | 97 : 3 | 3 | 8.000 |
| 5 | 90 : 10 | 3 | 5.500 |

## Patentansprüche

1. Verfahren zur Herstellung hellfarbiger, niedrigviskoser Alkyloligoglykoside, bei dem man Mischungen von
a) langkettigen Alkyloligoglucosiden der Formel (**I**),
**R**^{**1**}**O-[G]**_{**P**} **(I)**
in der R¹ für einen Alkylrest mit 16 bis 22 Kohlenstoffatomen, G für einen Zuckerrest mit 5 oder 6 Kohlenstoffatomen und p für Zahlen von 1 bis 10 steht, und
b) kurzkettigen Alkyloligoglucosiden der Formel **(II),**
**R**^{**2**}**O-[G]**_{**P**} **(II)**
in der R² für einen Alkylrest mit 8 bis 14 Kohlenstoffatomen, G für einen Zuckerrest mit 5 oder 6 Kohlenstoffatomen und p für Zahlen von 1 bis 10 steht,
bei einem pH-Wert im Bereich von 8-13 mit Peroxidverbindungen bleicht, wobei die Komponenten (a) und (b) im Gewichtsverhältnis 99 : 1 bis 40 : 60 eingesetzt werden.

2. Verfahren nach Anspruch 1, **dadurch gekennzeichnet, dass** man als Peroxidverbindung Wasserstoffperoxid einsetzt.

## Claims

1. A process for the production of light-colored, low-viscosity alkyl oligoglycosides, in which mixtures of
(a) long-chain alkyl oligoglucosides corresponding to formula **(I)**:
**R**^{**1**}**O-[G]**_{**p**} **(I)**
in which R¹ is an alkyl radical containing 16 to 22 carbon atoms, G is a sugar unit containing 5 or 6 carbon atoms and p is a number of 1 to 10, and
(b) short-chain alkyl oligoglucosides corresponding to formula **(II)**:
**(II): R**^{**2**}**O-[G]**_{**p**} **(II)**
in which R² is an alkyl radical containing 8 to 14 carbon atoms, G is a sugar unit containing 5 or 6 carbon atoms and p is a number of 1 to 10, are bleached with peroxide compounds at a pH value of 8 to 13, whereas components (a) and (b) are used in a ratio by weight of 99:1 to 40:60.

2. A process as claimed in claims 1, **characterized in that** hydrogen peroxide is used as the peroxide compound.

## Revendications

1. Procédé de préparation d'alkyloligoglycosides de couleur claire, peu visqueux, dans lequel des mélanges
a) d'alkyloligoglucosides à longue chaîne de formule (I)
R¹O-[G]_{P} (I)
dans laquelle R¹ représente un radical alkyle ayant de 16 à 22 atomes de carbone, G représente un reste de sucre ayant 5 ou 6 atomes de carbone et p représente un nombre allant de 1 à 10 et
b) d'alkyloligoglucosides à chaîne courte de formule (II)
R20-[G]p (II)
dans laquelle R2 représente un radical allyle ayant de 8 à 14 atomes de carbone, G représente un reste de sucre ayant 5 ou 6 atomes de carbone et p représente un nombre allant de 1 à 10,
sont blanchis avec des composés péroxydiques à une valeur de pH dans la zone de 8 à 13, les composants (a) et (b) étant mis en oeuvre dans un rapport pondéral de 99 : 1 à 40 : 60.

2. Procédé selon la revendication 1,
**caractérisée en ce que**
comme composé péroxydique on met en oeuvre du péroxyde d'hydrogène.
